# EUROPEAN PATENT APPLICATION

(11) **EP 1 792 618 A1**
(43) Date of publication of application: **06.06.2007**
(21) Application number: 05111527.7
(22) Date of filing: 30.11.2005
(51) Int. Cl.: A61K 31/55, A61P 29/02

(54) **R-mirtazapine for the treatment of pain**

(71) Applicant: Freynhagen, Rainer, 40225 Düsseldorf (DE)
(72) Inventor: Freynhagen, Rainer, 40225 Düsseldorf (DE)
(74) Representative: Broekkamp, Chris L. E.

(57) **Abstract**

This invention relates to a method of treatment of pain comprising administering to a recipient in need of treatment for pain a therapeutically effective dose of R-mirtazapine and the use of R-mirtazapine for the manufacture of a medicine for the treatment of pain and a pharmaceutical composition for the treatment of pain, comprising R-mirtazapine.

## Description

This invention relates to the use of a mirtazapine enantiomer for the treatment of pain.

Antidepressants have antinociceptive effects and are known to be used for the treatment of chronic pain (Ansari, Harv Rev Psychiatry, 7 (2000) 257-77; Carter and Sullivan, Curr Opin Investig Drugs, 3 (2002) 454-8; Reisner, Curr Pain Headache Rep, 7 (2003) 24-33, Saarto, Wiffen, Antidepressants for neuropathic pain. Cochrane Database Syst Rev. 2005 Jul 20;(3):CD005454). Especially tricyclic antidepressants (TCAs) are well established in the treatment of difficult conditions as neuropathic pain and tension type headache (Carter and Sullivan, Curr Opin Investig Drugs, 3 (2002) 454-8; Lynch, J Psychiatry and Neuroscience, 26 (2001) 30-36). However, as TCAs often have severe side effects and lack safety in overdose (Reisner, Curr Pain Headache Rep, 7 (2003) 24-33, Saarto, T., Wiffen, P. Antidepressants for neuropathic pain. Cochrane Database Syst Rev. 2005 Jul 20;(3):CD005454), a new generation of safer antidepressants with a better tolerability and fewer side effects is increasingly used to treat chronic pain (Ansari, Harv Rev Psychiatry, 7 (2000) 257-77). One of these novel antidepressive drugs is mirtazapine (Kelder et al., J Pharm Pharmacol, 49 (1997) 403-11) namely the racemic mixture (+/-)-mirtazapine. There are some clinical studies which suggest that (+/-)-mirtazapine might be useful in the treatment of pain in humans (Brannon and Stone, J Pain Symptom Manage, 18 (1999) 382-5; Bendtsen and Jensen, Neurology, 62 (2004) 1706-11; Theobald et al, J Pain Symptom Manage, 23 (2002) 442-7; Ansari, Harv Rev Psychiatry, 7 (2000) 257-77; Carter and Sullivan, Curr Opin Investig Drugs, 3 (2002) 454-8). But so far, only few experimental data exist (Bomholt et al., Neuropharmacology, 48 (2005) 252-63; Schreiber et al., Brain Res Bull, 58 (2002) 601-5; Schreiber et al., J Mol Neurosci, 18 (2002) 143-9) and none concerning the effects of its enantiomers. The serotonergic neurotransmission enhanced by an indirect and direct noradrenergic effect (Szegedi and Schwertfeger, Expert Opin Pharmacother, 6 (2005) 631-41) is possibly the basis of (+/-)-mirtazapines ability to block nociception (Schreiber et al., J Mol Neurosci, 18 (2002) 143-9; Schreiber et al., Brain Res Bull, 58 (2002) 601-5). However, there is evidence, that the enantiomers of (+/-)-mirtazapine, R(-)-mirtazapine and S(+)-mirtazapine, differ in their pharmacokinetic and pharmacodynamic properties (Gower et al., Arch Int Pharmacodyn Ther, 291 (1988) 185-201; De Boer, J Clin Psychiatry, 57 Suppl 4 (1996) 19-25; Haddjeri et al., Int Clin Psychopharmacol, 10 Suppl 4 (1995) 11-7; Haddjeri et al., J Pharmacol Exp Ther, 277 (1996) 861-71). In general it is known that stereochemistry of antidepressant and antipsychotic drugs is relevant for drug efficacy and development (Baker and Prior, Ann Med, 34 (2002) 537-43).

It has now been found that the efficacy of mirtazapine for the treatment of pain can be improved by treating with the R(-)-enantiomer only. The improvement is more than merely a reduction of the dose by selecting an active component out of the mixture of (+/-)-mirtazapine, but it amounts to removing a component with an hitherto unknown adverse contribution in the treatment results.

Contrary to the S(+)-mirtazapine, R(-)-mirtazapine exerts solely antinociceptive effects. The absence of S(+)-enantiomer removes a pronociceptive component from the racemic mirtazapine mixture, resulting in more effective and/or safer treatment of pain. With R(-)-mirtazapine, essentially free from the S(+)-enantiomer, a compound for antinociceptive treatment is provided of which the therapeutic window is larger than of that of (+/-)-mirtazapine . Thus, an R(-)-mirtazapine based treatment not only has higher efficacy, but also has less side effects or a larger range of dosages with less side effects in comparison to the use of racemic mirtazapine for the treatment of pain.

The meaning of the terms 'essentially free from S-enantiomer' may be expressed quantitatively by specifying a content of S-mirtazapine of less than 5%, 2%, 1 %, 0.5% or 0.1 % of the total content of mirtazapine.

An embodiment of this invention is the use of pure R(-)-mirtazapine for the manufacture of a medicament of the treatment of pain. Pure R-mirtazapine in this context means essentially free from S-mirtazapine.

Pain is a complaint, symptom or syndrome appearing in many forms and circumstances. The use according to the invention can be directed to counteracting acute or chronic pain entities, independent from the underlying pathophysiology as nociceptive, neuropathic or mixed pain syndrome. Examples can be pain in local parts of the body, in shoulders, back, low back or neck, myofacial pain, fibromyalgia syndromes, pain due to rheumatic diseases, inflammation, arthrosis or arthritis, post-operative pain, labor- and post-partum pain, post-traumatic pain, headache, tooth pain, visceral pain, cancer pain, psychogenic pain, neuropathic pain as polyneuropathy (e.g. diabetes, toxic, postherpetic, small fiber), deafferentation pain, phantom limb pain, trigeminal neuralgia, sympathetically maintained pain, complex regional pain syndromes Type I and II or central pain, e.g. pain associated with multiple sclerosis (MS) or stroke.

Therefore, it is a separate embodiment of the invention to use R(-)-mirtazapine for the treatment, or the manufacture of a medicine for that treatment, of chronic pain.

It is also a separate embodiment of the invention to use R(-)-mirtazapine for the treatment, or the manufacture of a medicine for that treatment, of nociceptive pain.

It is also a separate embodiment of the invention to use R(-)-mirtazapine for the treatment, or the manufacture of a medicine for that treatment, of neuropathic pain.

It is also a separate embodiment of the invention to use R(-)-mirtazapine for the treatment, or the manufacture of a medicine for that treatment, of mixed pain syndrome. It is also a separate embodiment of the invention to use R(-)-mirtazapine for the treatment, or the manufacture of a medicine for that treatment, of pain in local parts of the body, such as pain in shoulders, back, low back or neck.

It is also a separate embodiment of the invention to use R(-)-mirtazapine for the treatment, or the manufacture of a medicine for that treatment, of myofacial pain.

It is also a separate embodiment of the invention to use R(-)-mirtazapine for the treatment, or the manufacture of a medicine for that treatment, of pain due to fibromyalgia syndrome.

It is also a separate embodiment of the invention to use R(-)-mirtazapine for the treatment, or the manufacture of a medicine for that treatment, of post-operative pain.

It is also a separate embodiment of the invention to use R(-)-mirtazapine for the treatment, or the manufacture of a medicine for that treatment, of, pain due to rheumatic diseases, inflammation, arthrosis and arthritis.

It is also a separate embodiment of the invention to use R(-)-mirtazapine for the treatment, or the manufacture of a medicine for that treatment, of pain during labor.

It is also a separate embodiment of the invention to use R(-)-mirtazapine for the treatment, or the manufacture of a medicine for that treatment, of post-partum pain.

It is also a separate embodiment of the invention to use R(-)-mirtazapine for the treatment, or the manufacture of a medicine for that treatment, of post-traumatic pain.

It is also a separate embodiment of the invention to use R(-)-mirtazapine for the treatment, or the manufacture of a medicine for that treatment, of headache.

It is also a separate embodiment of the invention to use R(-)-mirtazapine for the treatment, or the manufacture of a medicine for that treatment, of tooth pain.

It is also a separate embodiment of the invention to use R(-)-mirtazapine for the treatment, or the manufacture of a medicine for that treatment, of visceral pain.

It is also a separate embodiment of the invention to use R(-)-mirtazapine for the treatment, or the manufacture of a medicine for that treatment, of pain due to cancer.

It is also a separate embodiment of the invention to use R(-)-mirtazapine for the treatment, or the manufacture of a medicine for that treatment, of psychogenic pain.

It is also a separate embodiment of the invention to use R(-)-mirtazapine for the treatment, or the manufacture of a medicine for that treatment, of neuropathic pain as polyneuropathy (e.g. diabetes, toxic, post-herpethic, small fiber).

It is also a separate embodiment of the invention to use R(-)-mirtazapine for the treatment, or the manufacture of a medicine for that treatment, of deafferentation pain.

It is also a separate embodiment of the invention to use R(-)-mirtazapine for the treatment, or the manufacture of a medicine for that treatment, of phantom limb pain.

It is also a separate embodiment of the invention to use R(-)-mirtazapine for the treatment, or the manufacture of a medicine for trigeminal neuralgia.

It is also a separate embodiment of the invention to use R(-)-mirtazapine for the treatment, or the manufacture of a medicine for that treatment, of sympathically maintained pain.

It is also a separate embodiment of the invention to use R(-)-mirtazapine for the treatment, or the manufacture of a medicine for that treatment, of complex regional pain syndromes Type I and II.

It is also a separate embodiment of the invention to use R(-)-mirtazapine for the treatment, or the manufacture of a medicine for that treatment, of central pain. e.g. pain associated with multiple sclerosis (MS) or stroke.

R-mirtazapine can be used for the purpose according to the invention as a free base or as one or more of the commonly accepted acid addition salts. Such compounds can be used in pure form or in admixture with pharmaceutical excipients. Preferred forms of mirtazapine are those in salt form which will result in stable pharmaceutical formulations.

The meaning of pure as characteristic of pure R-mirtazapine refers to the enantiomeric purity of the total active mirtazapine ingredient in the medicine according to the invention. Purity means that the therapeutic action of mirtazapine in the medicine is based on the action of the R-mirtazapine component in the medicine, without contribution from the S-enantiomer in conteracting the symptoms of hot flush. This would be the situation if more that 80% is of the total mirtazapine content of the medicine is R-mirtazapine, although higher purity is preferred, such as at least 90%, or 95%, or 99%, or 99.5% of total mirtazapine content.

The amount of R-mirtazapine, also referred to herein as the active ingredient, which is required to achieve a therapeutic effect will, of course, vary with the particular compound, the route of administration and the age and other conditions of the recipient.

The amounts of mirtazapine defined in this description refer to the amount of free base of mirtazapine, unless indicated otherwise.

A suitable daily dose will be in the range of 0.5 to 140 mg, calculated on the weight content of base, per recipient per day, preferably in the range of 1 to 25 mg of the base per recipient per day. In general, parenteral administration requires lower dosages than other methods of administration which are more dependent upon absorption. However, the daily dosages are between 0.01 and 2 mg/kg body weight of the recipient. The recipient is the woman, man or male or female animal receiving the dose of R-mirtazapine for treatment of pain.

In the case of tolerance development, treatments can be further optimized by increasing the dose up to 5 times the initial dose in the course of a chronic treatment in humans. The desired daily dose may be presented as one, two, three or more sub-doses administered at appropriate intervals throughout the day. A treatment may be for a single day, at discretion of the patient on an "if needed" basis or for a limited determined treatment period defined by a number of days, weeks or months.

While it is possible for the active ingredient to be administered alone, it is preferable to present it as a pharmaceutical formulation. Accordingly, the present invention further provides a pharmaceutical formulation for use in the treatment of pain comprising pure R-mirtazapine, together with a pharmaceutically acceptable carrier thereof and optionally other therapeutic agents. The carrier must be "acceptable" in the sense of being compatible with the other ingredients of the formulation and not deleterious to the recipients thereof. Suitable excipients are made available e.g., in the Handbook of Pharmaceutical Excipients, 2nd Edition; Editors A. Wade and P.J.Weller, American Pharmaceutical Association, Washington, The Pharmaceutical Press, London, 1994. The invention further includes a pharmaceutical formulation, as hereinbefore described, in combination with packaging material suitable for the pharmaceutical formulation, said packaging material including instructions for the use of the pharmaceutical formulation in the treatment of pain.

Formulations include those suitable for oral or rectal administration. The formulations may be prepared by any methods well known in the art of pharmacy, for example, using methods such as those described in Gennaro et al., Remmington: The Science and Practice of Pharmacy, 20th Edition, Lippincott, Williams and Wilkins, 2000; see especially part 5: pharmaceutical manufacturing.. Such methods include the step of bringing into association the active ingredient with the carrier which constitutes one or more accessory ingredients. Such accessory ingredients include those conventional in the art, such as, fillers, binders, diluents, disintegrants, lubricants, colorants, flavoring agents and wetting agents.

Formulations suitable for oral administration may be presented as discrete units such as tablets or capsules each containing a predetermined amount of active ingredient; as a powder or granulates; as a solution or suspension. The active ingredient may also be presented as a bolus or paste, or may be contained within liposomes or microparticles.

Formulations, which are parenteral (for example subcutaneous) may also be presented in a suitable sustained release form.

R-mirtazapine can be prepared in several manners, e.g. by purification from the racemic mixture mirtazapine. Mirtazapine may be prepared using the method described in US 4,062,848. R-mirtazapine can also be obtained by stereoselective synthesis (W0200541 0).

The following example is for illustration and should not be considered to be limiting in anyway:

### Example

The pro- or antinociceptive properties of (+/-)-mirtazapine and its enantiomers R(-)-mirtazapine and S(+)mirtazapine, respectively, were analyzed in acute thermal pain (Hargreaves et al., Pain, 32 (1988) 77-88). Male Wistar rats weighing 350-400 g (housed three per cage, access to foot and water ad libitum, room maintained at 22°C ± 0,5°C and on a 12:12 light- dark cycle) were anesthetized with intraperitoneal (i.p.) injection of pentobarbital (60 mg/kg i.p.). The rats were implanted with polyethylene catheters (inner diameter 0.28 mm, outer diameter 0.61 mm), which were advanced from the cisterna magna to the rostral edge of the lumbar enlargement as described before (Muth-Selbach et al., Anesthesiology, 101 (2004) 753-8; Ahmadi et al., Science, 300, (2003) 2094-2097; Zeilhofer et al., J Neurosci 20 (2000) 4922-9). Rats with impaired motor function after catheter implantation were excluded from the study. Paw withdrawal latencies were tested 6-10 days after surgery at all times in the same air conditioned room. The lateral plantar surface of both hind paws was exposed to a defined radiant heat stimulus through a transparent perspex surface (Plantar Test, Hugo Basile Inc., Italy, Comerio) and paw withdrawal latencies were recorded (Hargreaves et al., op.cit.). The animal is unrestrained and unhandled during experiments. When the rat feels pain and withdraws its paw, the sudden drop of reflected radiation switches off the I.R. source and stops the reaction time counter so that the withdrawal latency to the nearest 0.1 s is determined. Paw withdrawal latencies of both hind paws were determined from 4 independent measurements for each time point. A cut-off time of 20 sec was set to avoid tissue damage.

Behavior as described above was tested before and 120, 240 min and 24 hours after application of either substance. In order to find the maximum effect over time, groups of 4 animals were tested before and 30, 60, 120, 180, 240 min and 24 hours after application of 0.1 mg/kg (+/-)-mirtazapine.

(+/-)-Mirtazapine, R(-)-mirtazapine, S(+)mirtazapine or vehicle (0.9% NaCl) were applied to the cerebrospinal fluid via intrathecal catheters in a total volume of 10 µl. Rats were randomly assigned to the different treatment groups consisting of 6 to 9 rats each. All behavioral observations were performed in a standardized blinded fashion.

After the behavioral tests, rats were sacrificed by a lethal dose of pentobarbital i.p.. Proper position of the catheter tip was verified after laminectomy and methylene blue injection through the catheter. All behavioral tests were performed with permission of the German authorities concerned with the law on experiments involving animals and were in accordance with the guidelines of the International Association for the Study of Pain.

### Chemicals

(+/-)-Mirtazapine (1, 2, 3, 4, 10, 14b-Hexahydro-2-methyl-pyrazino-[2, 1-a] pyrido [2, 3-c] [2] benzazepin, free base), R(-)-mirtazapine and S(+)-mirtazapine maleate salt were generously donated by N.V. Organon, Oss (The Netherlands) and were of the highest quality available. All chemicals were dissolved in 0.9 % NaCl and stored in aliquots at - 70°C. Fresh dilutions were made with 0.9 % NaCl on every experimental day.

### Statistical analysis

Group data are presented as mean + standard error of the mean(sem). Withdrawal latencies in the different treatment groups were analyzed by analysis of variance (repeated measurements) followed by LSD post hoc tests (SAS Software System). P ≤ 0.05 was assumed significant. In detail, for each dose (i.e. 1, 0.1, 0.01 and 0.001 mg/kg) twelve comparisons were made at five time points.

### Results

Because the effects at 30, 60 and 180 min after application of (+/-)-mirtazapine added no further information, the measurements at those time points were omitted in the final results.

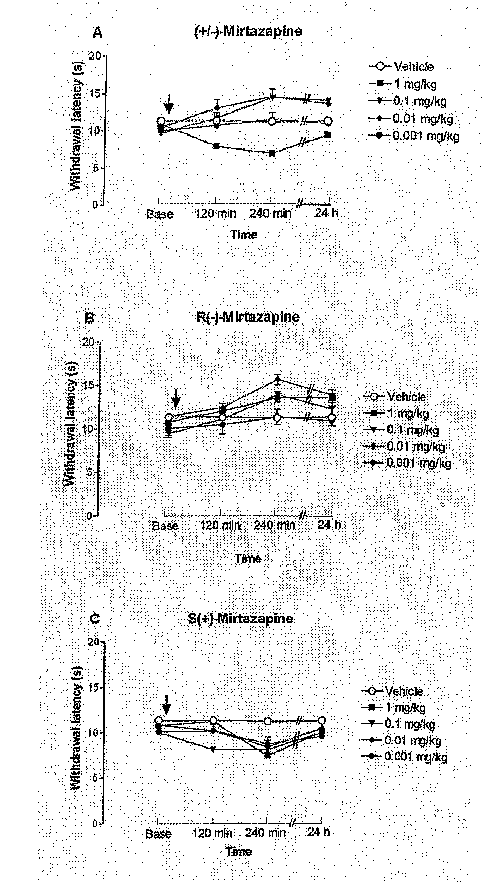

When injected intrathecally (+/-)-mirtazapine dose-dependently exerted pro- or antinociceptive effects in acute thermal pain. At lower doses (0.1 and 0.01 mg/kg), (+/-)-mirtazapine was antinociceptive while the highest dose (1 mg/kg) elicited a profound pronociceptive effect at 120, and at 240 minutes as well as 24 hours after intrathecal administration. At 240 minutes after injection the maximum of pro- or antinociception was observed. The lowest dose (0.001 mg/kg) was neither pro- nor antinociceptive. Paw withdrawal latencies at 240 minutes after intrathecal injection were 6.9 ± 0.4 s (1 mg/kg), 14.4 ± 0.3 s (0.1 mg/kg), 14.5 ± 1.0 s (0.01 mg/kg) and 11.5 ± 0.9 s (0.001 mg/kg) versus 11.2 ± 0.24 s (vehicle) (fig. 1; p < 0.001 for 1, 0.1 and 0.01 mg/kg vs vehicle and not statistically significant for 0.001 mg/kg vs vehicle; n = 6 to 9 rats per group).

At doses of 1, 0.1 and 0.01 mg/kg, R(-)-mirtazapine was antinociceptive at 240 minutes and 24 hours after intrathecal administration and elicited no pronociception at either dose. At 240 minutes after injection the maximum of antinociception was observed. Again the lowest dose (0.001 mg/kg) was neither pro- nor antinociceptive. Paw withdrawal latencies 240 minutes after intrathecal injection were 13.5 ± 0.5 s (1 mg/kg), 13.8 ± 0.4 s (0.1 mg/kg), 15.6 ± 0.6 s (0.01 mg/kg) and 11.3 ± 0.9 s (0.001 mg/kg) versus 11.2 ± 0.24 s (vehicle) (fig. 1; p < 0.001 for 1, 0.1 and 0.01 mg/kg vs vehicle and ns for 0.001 mg/kg vs vehicle; n = 6 to 9 rats per group).

In contrast, S(+)-mirtazapine was pronociceptive 120 and 240 minutes and 24 hours (0.1 mg/kg), 240 minutes and 24 hours (1 mg/kg) and 240 minutes (0.01 mg/kg) after intrathecally administration and elicited no antinociception at either dose. Again 240 minutes after intrathecal injection the maximum of the pronociceptive effect was observed and the lowest dose (0.001 mg/kg) of S(+)-mirtazapine was neither pro- nor antinociceptive (fig. 1, there was only a tendency towards decreased paw withdrawal latencies 240 minutes after intrathecal injection, p = 0.0507, n = 6). Paw withdrawal latencies were 7.4 ± 0.4 s (1 mg/kg), 8.1 ± 1.0 s (0.1 mg/kg), 8.4 ± 1.1 s (0.01 mg/kg) and 8.7 ± 0.8 s (0.001 mg/kg) versus 11.2 ± 0.24 s (vehicle) 240 minutes after intrathecal injection (fig.1; p < 0.001 for 1, 0.1 and 0.01 mg/kg vs vehicle and ns for 0.001 mg/kg vs vehicle; n = 6 to 9 rats per group).

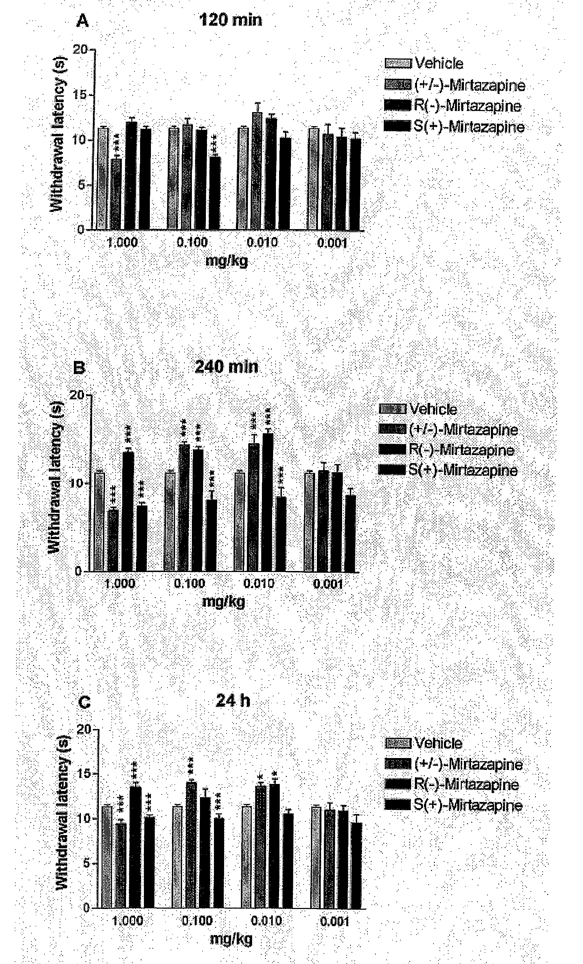

In comparison, (+/-)-mirtazapine, R(-)-mirtazapine and S(+)-mirtazapine had profound differential effects while modulating acute thermal pain (fig. 2). Whereas (+/-)-mirtazapine dose-dependently exerted pro- and- antinociceptive activity, S(+)-mirtazapine was exclusively pronociceptive at all doses tested. In contrast R(-)-mirtazapine only elicited antinociceptive effects at the wide dose range tested. Additionally, R(-)-mirtazapine resembled the action of (+/-)-mirtazapine in moderate doses (fig.1 and fig.2).

### Conclusion and discussion

Intrathecal application of (+/-)-mirtazapine dose-dependently increased the nociceptive threshold in this validated rat model of acute thermal pain in agreement with previously published observations after systemic (intraperitoneal) administration of (+/-)-mirtazapine in animals (Schreiber et al., Brain Res Bull, 58 (2002) 601-5; Schreiber et al., J Mol Neurosci, 18 (2002) 143-9; Bomholt et al., Neuropharmacology, 48 (2005) 252-63). The validity of animal models for pain is discussed in Le Bars et al., 53 (2001) 597-652). Thus, (+/-)-mirtazapine (3-30 mg/kg intraperitoneally) exhibited antinociceptive activity in thermal pain in rats (hot plate) (Bomholt et al., Neuropharmacology, 48 (2005) 252-63). In the chronic constriction injury model of neuropathic pain (+/-)-mirtazapine significantly attenuated thermal hyperalgesia and mechanical hyperalgesia but not mechanical allodynia in rats (Bomholt et al., Neuropharmacology, 48 (2005) 252-63). In contrast, (+/-)-mirtazapine did not affect acute nociceptive responses in rats in the tail flick test.

Not surprisingly, even the biphasic dose-response curve as described by Schreiber et al. (Brain Res Bull, 58 (2002) 601-5) after intraperitoneal injection of (+/-)-mirtazapine in mice was observed. After intrathecal injection of 0.1 and 0.01 mg/kg (+/-)-mirtazapine nociceptive thresholds increased, 0.001 mg/kg did not change nociceptive thresholds and 1 mg/kg decreased nociceptive thresholds implying a clear "therapeutic window effect" as already suggested Schreiber et al., Brain Res Bull, 58 (2002b) 601-5 (fig. 1). So far, no reason for this biphasic effect is known or has been suggested.

In contrast to (+/-)-mirtazapine, after intrathecal application of S(+)-mirtazapine or R(-)-mirtazapine no biphasic dose-response curve was observed. Thus, R(-)-mirtazapine was solely antinociceptive and S(+)-mirtazapine, surprisingly, pronociceptive.

As S(+)-mirtazapine is the more potent blocker of α₂-adrenergic autoreceptors than R(-)-mirtazapine, it should have the greater effect on enhancing noradrenergic neurotransmission and the greater effect on serotonergic neurotransmission. Consequently, it should also have the greater antinociceptive effect. Contrarily and surprisingly, S(+)-mirtazapine exerted pronociception in the tested dose range for at least 24 hours after acute intrathecal injection. Similar results have been obtained by McGrath et al. (Eur J Pharmacol, 356 (1998) 121-6). Here, (+/-)-mirtazapine potentiated the hypothermic response to a 5-HT₁-agonist to a greater extent than one of the enantiomers alone (McGrath et al., Eur J Pharmacol, 356 (1998) 121-6), implying that none of the enantiomers appear to be more active concerning serotonergic neurotransmission than the racemic mixture in normal rats after chronic administration (McGrath et al., Eur J Pharmacol, 356 (1998) 121-6).

Several clinical studies indicate that antidepressants which selectively affect only the adrenergic or the serotonergic system may be less efficacious than drugs affecting both pathways to a similar extent (Briley, 4 (2003) 42-45; Burke, J Clin Psychiatry, 65 Suppl 4 (2004) 37-45; Fishbain et al., Pain Med, 1 (2000) 310-316; McQuay et al., Pain, 68 (1996) 1080-192; Otsuka et al., J Psychopharmacol, 12 (2001) 407-413; Owens, J Clin Psychiatry, 65 (suppl. 4) (2004) 5-10; Smith, 12 (1998) 407-413; Staiger et al., Spine, 28 (2003) 2540-2545). Hence, the potency of the individual enantiomer of (+/-)-mirtazapine in blocking α₂-adrenergic autoreceptors might not be the critical point for its antinociceptive activity but the overall effect on both, noradrenergic and serotonergic neurotransmission.

This work was supported by grants from the Forschungskommission der Heinrich Heine Universität Düsseldorf to U.M.S.

### Figure legends

Fig. 1: Dose-dependent effects of (+/-)-mirtazapine (A), R(-)-mirtazapine (B) and S(+)-mirtazapine (C) on acute thermal pain in rats. Paw withdrawal latencies in reponse to thermal stimulation (mean ± sem) (in s) of rats treated with different doses of (+/-)-mirtazapine, R(-)-mirtazapine and S(+)-mirtazapine injected intrathecally versus time. 0.001, 0.01, 0.1 and 1 mg/kg and vehicle. Arrow indicates time of drug injection. n = 6 to 9 rats per group.

Fig. 2: Comparison of the effects of (+/-)-mirtazapine, R(-)-mirtazapine and S(+)-mirtazapine on acute thermal pain in rats. Paw withdrawal latencies in reponse to thermal stimulation (mean ± sem) (in s) of rats treated with different doses (0.001, 0.01, 0.1 and 1 mg/kg) of (+/-)-mirtazapine, R(-)-mirtazapine, S(+)-mirtazapine and vehicle injected intrathecally. (A) 120 min, (B) 240 min and (C) 24 hours after drug injection. ***P< 0.001, *P<0.05 versus vehicle-treated animals. n = 6 to 9 rats per group.

## Claims

1. A method of treatment of pain comprising administering to a recipient in need of treatment for pain a therapeutically effective dose of R-mirtazapine.

2. The use of R-mirtazapine for the manufacture of a medicine for the treatment of pain.

3. A pharmaceutical composition for the treatment of pain, comprising pure R-mirtazapine as active ingredient.
